# EUROPÄISCHE PATENTSCHRIFT

(11) **EP 1 305 070 B1**
(45) Veröffentlichungstag und Bekanntmachung des Hinweises auf die Patenterteilung: **07.12.2005**
(21) Anmeldenummer: 01960364.6
(22) Anmeldetag: 22.06.2001
(51) Int. Cl.: A61M 16/06, A62B 18/08, A41D 20/00

(54) **HALTERUNG FÜR EINE ATEMMASKE**
HOLDING DEVICE FOR A RESPIRATORY MASK
SYSTEME DE RETENUE POUR MASQUE RESPIRATOIRE

(30) Priorität: 21.07.2000 DE 10035946
(43) Veröffentlichungstag der Anmeldung: 02.05.2003
(73) Patentinhaber: MAP Medizin-Technologie GmbH, 82152 Martinsried (DE)
(72) Erfinder: VÖGELE, Harald, 82131 Gauting (DE); MADAUS, Stefan, 82152 Krailling (DE)
(74) Vertreter: Rössig, Rolf
(86) Internationale Anmeldenummer: PCT/EP2001/007132
(87) Internationale Veröffentlichungsnummer: WO 2002/007806

(56) Entgegenhaltungen:
- EP-A- 0 958 841
- DE-U- 29 923 126
- US-A- 3 792 702
- US-A- 4 910 804
- US-A- 5 687 715

## Beschreibung

Die Erfindung betrifft eine Halterung für eine Atemmaske, wie sie beispielsweise im Bereich der Schlafmedizin zur Fixierung einer Nasalmaske im Gesicht des Patienten Anwendung finden kann. Eine solche Atemmaske ist beispielsweise in EP-A-0 958 841 beschrieben.

Bekannte Halterungen der eingangs genannten Art umfassen üblicherweise eine obere Gurtanordnung und eine untere Gurtanordnung, die über eine in Applikationsposition der Halterung im Hinterkopfbereich des Patienten befindliche Steganordnung miteinander verbunden sind. Die beiden Gurtbandanordnungen sind aus einem flexiblen Textilmaterial gefertigt. Im Bereich der freien Endabschnitte der oberen, bzw. der unteren Gurtanordnung sind Klettverschlußeinrichtungen vorgesehen, durch welche die wirksame Länge und damit der Anpressdruck der Atemmaske gegen das Gesicht des Patienten bedarfsgerecht eingestellt werden kann. Die obere Gurtanordnung kann bei bestimmten Maskentypen mit einem Stirnhalter verbunden werden, so dass durch Einstellung der wirksamen Länge der oberen Gurtanordnung der Anpressdruck des Stirnauflageelementes gegen die Stirn des Patienten festgelegt werden kann.

In Abhängigkeit von dem erforderlichen Therapiedruck des über die Atemmaske zugeführten Atemgases sowie auch in Abhängigkeit von der individuellen Gesichtstektur des Patienten sind teilweise Maskenanpresskräfte erforderlich, die nach längerer Applikation der Maske im Gesicht des Patienten oder auch im Stirnbereich deutlich sichtbare Druckstellen verursachen.

Der Erfindung liegt die Aufgabe zugrunde, eine einfach handhabbare Halterung für eine Atemmaske zu schaffen, durch welche eine geforderte Atemmaskenanpresskraft unter einem verbesserten Tragekomfort auf zuverlässige Weise aufgebracht werden kann.

Diese Aufgabe wird erfindungsgemäß gelöst durch eine Halterung für eine Atemmaske mit den in Patentanspruch 1 angegebenen Merkmalen.

Dadurch wird es auf vorteilhafte Weise möglich, die Atemmaske bei geringen Spannkräften zugsteif auf dem Gesicht des Patienten zu fixieren. Für den Fall, dass beispielsweise aufgrund eines erhöhten Maskeninnendrucks die Maske dazu tendiert, sich vom Gesicht des Patienten abzuheben, ergibt sich selbsttätig eine entsprechend größere Maskenfixierkraft.

In vorteilhafter Weise wird eine extrem zuverlässige Fixierung des Stirnauflageelementes im Stirnbereich des Patienten erreicht, ohne dass erhebliche Zugkräfte an der sich über Stirn und Hinterkopf erstreckenden Gurtbandanordnung ergeben.

Eine im Hinblick auf einen besonders hohen Tragekomfort vorteilhafte Ausführungsform der Erfindung ist dadurch gegeben, dass die obere Gurtbandanordnung derart tailliert ausgebildet ist, dass sich diese vom Stirnbereich ausgehend zunächst zum jeweiligen oberen Ohrbereich des Patienten hin erstreckt, dann im Bereich der Ohren einen nach oben zum Scheitel hin ausweichenden Verlauf einnimmt und unmittelbar hinter den Ohren nach unten abfällt und den Hinterkopfbereich des Patienten im Bereich des Nackens oder etwa auf Höhe der Nase des Patienten umgreift.

Die in der Halterung vorgesehene flexible Einlage kann beispielsweise aus einer 0,8 - 1,5 mm dicken thermoplasitschen Kunststoffmateriallage gefertigt sein. In einem in Applikationsposition zwischen der zugsteifen Einlage und dem Patienten vorgesehenen Bereich befindet sich vorzugsweise eine Polsterauflage. Diese Polsterauflage ist gem. einer besonders bevorzugten Ausführungsform der Erfindung durch eine dünne und lokal abgesteppte Schaumstofflage gebildet, auf deren Außenseite ein Bahnmaterial aufkaschiert ist. Bei diesem Bahnmaterial kann es sich um ein textiles Material oder auch um ein beispielsweise nass zu reinigendes, abwaschbares Material handeln.

Die Stirnbandanordnung der Atemmaske ist vorzugsweise mit einer Einstelleinrichtung versehen, durch welche die wirksame Länge des Stirnbandes stufenlos einstellbar ist. Die Verstelleinrichtung kann durch einen Rast-/Klemm-Verschiebemechanismus und/oder auch durch eine Klettverschlußeinrichtung gebildet sein.

Gemäß einer besonders bevorzugten Ausführungsform der Erfindung ist die flexible Lage aus einem Kunststoffmaterial, beispielsweise einem thermoplastischen Kunststoffmaterial gebildet. Alternativ hierzu oder auch in Kombination damit, ist es auch möglich, die flexible Lage aus einem Metallmaterial, beispielsweise einem dünnen Federstahlblech zu bilden. Die Biegeelastizität der flexiblen Lage kann durch Ausstanzungen oder Tiefziehstrukturen definiert beeinflusst werden. Die flexible Lage ist gemäß einer besonders bevorzugten Ausführungsform der Erfindung aus einem entsprechenden Bahnmaterial durch einen Stanzvorgang ausgestanzt. Alternativ dazu ist es auch möglich, die flexible Lage durch einen Kunststoffformvorgang, beispielsweise aus einem thermoplastischen Kunststoff in einem Spritzwerkzeug zu fertigen. Hierbei ist es möglich, besondere versteifende oder funktionale Strukturen an der Maskenhalterung auszubilden.

Gemäß einer besonders bevorzugten Ausführungsform der Erfindung ist die flexible Lage aus einem thermo-anformbaren Material gebildet. Dadurch wird es auf vorteilhafte Weise möglich, das beispielsweise auf eine Temperatur von 60° C erwärmte Stirnband individuell an den Patienten anzupassen, wobei das Stirnband nach Abkühlen auf Umgebungstemperatur eine im Hinblick auf eine möglichst geringe Flächenpressung vorteilhafte räumliche Gestalt beibehalten kann.

Eine im Hinblick auf einen besonders hohen Tragekomfort vorteilhafte Ausführungsform der Erfindung ist dadurch gegeben, dass der Stirnbandabschnitt mit einer Polstereinrichtung versehen ist. Die Polstereinrichtung kann vorzugsweise aus einem offenzellig geschäumten Schaumstoffmaterial gebildet sein. Die Polstereinrichtung ist vorzugsweise mit einer textilen oder abwaschbaren Lage versehen. Durch die Ausbildung lokal abgesteppter Abschnitte kann das Polsterverhalten der Polstereinrichtung in vorteilhafter Weise abgestimmt werden.

Eine im Hinblick auf eine besonders zuverlässige Fixierung der Atemmaske an dem Stirnband vorteilhafte Ausführungsform der Erfindung ist dadurch gegeben, dass die Halterung zumindest abschnittsweise mit einer der beiden Komplementärstrukturen einer Klettverschlußeinrichtung - vorzugsweise einem Vliesmaterial - versehen ist. Dazu ist das Stirnband vorzugsweise mehrlagig kaschiert ausgebildet. So kann beispielsweise die flexible, ggf. mit Durchbrüchen versehene Lage den Kernbereich des Stirnbandes bilden, wobei auf der, in Applikationsposition des Stirnbandes den Kopf des Patienten zugewandten Seite, eine Polsterung und eine hinsichtlich Struktur und Farbe ausgewählte Decklage aufkaschiert sind.

Eine im Hinblick auf einen besonders hohen Tragekomfort vorteilhafte Ausführungsform der Erfindung ist dadurch gegeben, dass der Stirnbandabschnitt in Applikationsposition eine vom Stirnbereich zum oberen Ohrbereich gerichteten Verlauf und einen im Ohrbereich lokal hochgezogenen Verlauf aufweist, wobei unmittelbar nach dem Ohrbereich der Stirnbandabschnitt einen zum Hinterkopf abfallenden Verlauf einnimmt.

Die eingangs angegebene Aufgabe wird gemäß einem weiteren Lösungsgedanken aufgelöst durch eine Halterung für eine Atemmaske mit einem sich vom Stirnbereich ausgehend seitlich zum oberen Ohrbereich hin erstreckenden Element erhöhter Biegesteifigkeit und einem hinter dem Ohrbereich zum Nacken hin abfallenden Bügelabschnitt sowie einer Einrichtung zum Abstützen des Bügelabschnittes im Hinterkopfbereich.

Dadurch wird es auf vorteilhafte Weise möglich, eine Atemmaske auf komfortable Weise zu applizieren und ggf. auf eine untere Gurtbandanordnung zu verzichten.

Das Element erhöhter Biegesteifigkeit kann beispielsweise aus einem Drahtmaterial, insbesondere aus einem Federstahlmaterial gefertigt sein. In vorteilhafter Weise weist das Element erhöhter Biegesteifigkeit einen zur Nasenspitze des Patienten vordringenden Abschnitt auf, der die Atemmaske gegen das Gesicht des Patienten drängt.

Die Einleitung der Kräfte in die Atemmaske erfolgt vorzugsweise derart, dass eine Kraftwirkungslinie durch einen Kräfteschwerpunkt der Maskenauflagefläche verläuft.

Die Abstützung am Hinterkopf erfolgt vorzugsweise durch ein gepolstertes Bandelement derart, dass durch die Abstützung eine Kraftwirkungslinie definiert wird, die mit der auf die Maske wirkenden Kraftwirkungslinie weitgehend übereinkommt.

Weitere Einzelheiten ergeben sich aus der nachfolgenden Beschreibung in Verbindung mit der Zeichnung. Es zeigen:
- Fig. 1: eine vereinfachte Darstellung zur Erläuterung der Applikafionsposifion einer Halterung für eine Atemmaske gem. einer ersten bevorzugten Ausführungsform der Erfindung;
- Fig. 2: eine vereinfachte Schnittansicht zur Erläuterung eines bevorzugten Innenaufbaus einer Halterung für eine Atemmaske mit einer seitlich biegesteifen flexiblen Einlage;
- Fig. 3: eine weitere, vereinfachte Schnittansicht zur Erläuterung des Innenaufbaus einer weiteren bevorzugten Ausführungsform der Halterung für eine Atemmaske, ebenfalls mit einer flexiblen Einlage;
- Fig. 4: eine vereinfachte Schnittansicht durch eine Halterung für eine Atemmaske gem. einer vierten bevorzugten Ausführungsform mit einer durch eine Steppnaht abgesteppten, gepolsterten Umfangskante;
- Fig. 5: eine perspektivische Ansicht eines Abschnitts einer Halterung für eine Atemmaske mit einer in Seitenrichtung biegesteifen flexiblen Einlage sowie integrierter Polstereinrichtung;
- Fig. 6: eine vereinfachte Detail-Schnittansicht zur Erläuterung der durch einen Schmelz-Schweißvorgang ausgebildeter Steppstellen auf der Innenseite des Stirnbandes der Halterung;
- Fig. 7: eine perspektivische Ansicht einer weiteren Ausführungsform einer Halterung für eine Atemmaske, hier mit einem Bügelelement zur Aufbringung einer Atemmaskenanpresskraft;
- Fig. 8: eine vereinfachte perspektivische Ansicht einer weiteren Ausführungsform einer Halterung für eine Atemmaske mit einer Versteifungseinlage;
- Fig. 9: eine vereinfachte Ansicht der Halterung, im wesentlichen jedoch mit integrierten Stirnauflageelement-Befestigungslaschen gem. Fig. 8 in Applikationsposition;

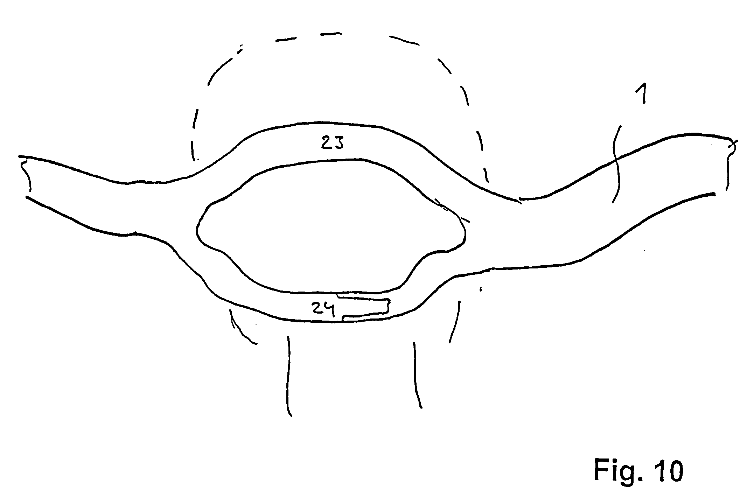

- Fig. 10: eine vereinfachte Darstellung zur Erläuterung einer weiteren Ausführung einer Halterung einer Atemmaske mit einer flexiblen Versteifungseinlage und einer hier in Applikationsposition im Hinterkopfbereich ausgebildeten Öffnung;
- Fig. 11: eine Prinzipskizze zur Erläuterung der Biegesteifigkeit der flexiblen Einlage, um deren Hauptachsen.

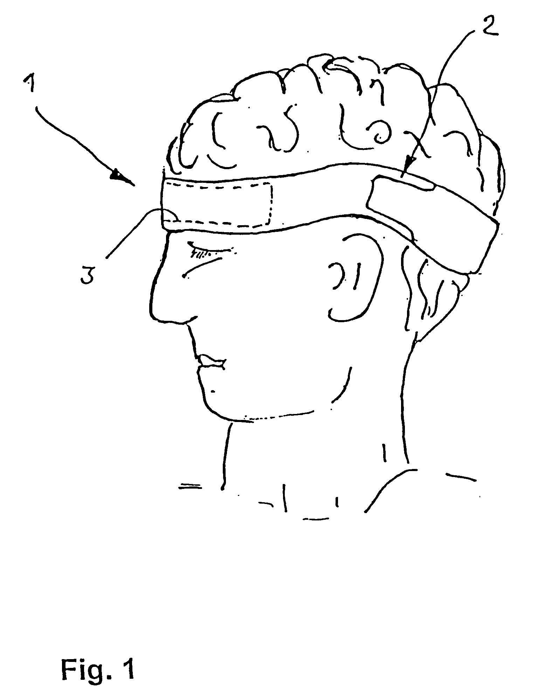

In Fig. 1 ist eine erste bevorzugte Ausführungsform einer Halterung für eine Atemmaske dargestellt, die hier einen Stirnbandabschnitt 1 aufweist, der mit einer in Abwicklungsrichtung flexiblen Lage seitlich ausgesteift ist. Der Stirnbandabschnitt 1 erstreckt sich in Applikationsposition vom Stirnbereich des Patienten ausgehend um den Hinterkopfbereich des Patienten herum. Zur Anpassung der wirksamen Länge des Stirnbandabschnitts 1 an den individuellen Kopfumfang des Patienten ist eine Einstelleinrichtung 2 vorgesehen, die bei der hier dargestellten Ausführungsform durch eine Klettverschlussverbindungseinrichtung gebildet ist. Der hier sichtbare Außenbereich des Stirnbandabschnitts 1 ist durch ein Vliesmaterial gebildet, das mit entsprechend komplementären Klettverschlussstrukturen in Haftverbindung bringbar ist. Hierdurch wird es möglich, insbesondere in dem durch Strichlinien 3 angedeuteten Bereich ein Stirnauflageelement einer Atemmaske zu fixieren. Durch die in das Stirnband integrierte flexible Lage ist es möglich, dem Stirnband in Seitenrichtung einen willkürlichen Verlauf zu geben. Bei der hier dargestellten Ausführungsform erstreckt sich das Stirnband vom vorderen Stirnbereich zunächst zum oberen Ohrbereich hin und weist oberhalb des Ohres einen nach oben ausweichenden Verlauf auf. In seinem weiteren Verlauf zum Hinterkopfbereich fällt das Stirnband wieder zum Nacken hin ab.

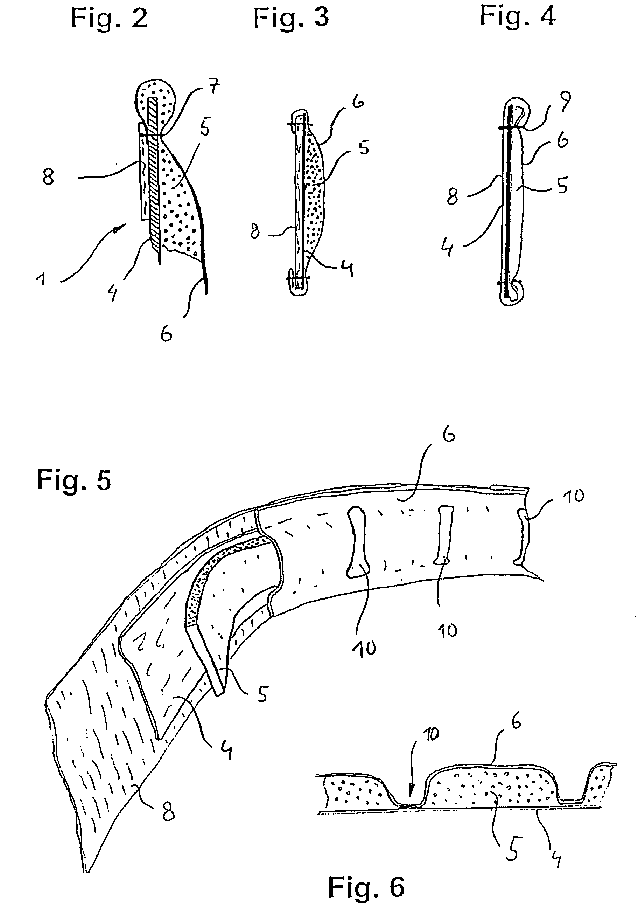

In Fig. 2 ist eine vereinfachte Schnittansicht durch einen Abschnitt eines Stirnbandes 1 dargestellt.

Das Stirnband 1 weist eine hier aus einem thermoplastischen Kunststoffmaterial gebildete Versteifungseinlage 4 auf, deren Dicke hier 0,75 mm beträgt. Auf der in Applikationsposition dem Patienten zugewandten Seite der Versteifungseinlage 4 ist eine Polsterung 5 vorgesehen, die hier aus einem offenzellig geschäumten Schaumstoffmaterial gebildet ist. Die Polsterung 5 wiederum ist abgedeckt durch eine hier aus einem Textilmaterial gefertigte Decklage 6. Die Decklage 6 sowie die Polsterung 5 sind über eine Verbindungsstelle 7 mit der flexiblen Lage 4 gekoppelt. Die Verbindungsstelle 7 ist hier durch eine Steppnaht gebildet.

Die Polsterung 5 sowie ein Abschnitt der Decklage 6 sind über eine Seitenkante der Versteifungseinlage 4 herumgeführt. Hierdurch wird eine besonders vorteilhafte Polsterung der Seitenkante der Versteifungseinlage 4 erreicht. Der um die Versteifungseinlage 4 herumgeführte Abschnitt ist durch die genannte Steppnaht wiederum auf die Versteifungseinlage 4 aufgenäht. Die Steppnaht erstreckt sich bei der hier dargestellten Ausführungsform weiterhin durch ein Vliesmaterial 8, das in Applikationsposition des Stirnbandes nach außen weist. Das Vliesmaterial 8 ist zusätzlich unmittelbar auf die Versteifungseinlage 4 aufgeklebt.

In Fig. 3 ist eine weitere Ausführungsform des Stirnbandes dargestellt. Hier ist der Polsterkörper 5 im Rahmen eines Flammkaschiervorganges auf die Versteifungseinlage 4 aufkaschiert. Auf der Rückseite der Versteifungseinlage 4 ist ähnlich wie bei der Ausführungsform gem. Fig. 2 eine Vliesmateriallage 8 vorgesehen. Die Vliesmateriallage 8 ist hinsichtlich ihrer Schichtdicke vorzugsweise derart ausgebildet, dass auch nach außen hin durch die Vliesmateriallage 8 ein ausreichender Polstereffekt erreicht wird. Bedarfsweise kann auch zwischen der Vliesmateriallage 8 und der Versteifungseinlage 4 eine Polsterung vorgesehen sein. Bei der in Fig. 3 dargestellten Ausführungsform sind die Seitenkanten der Versteifungseinlage 4 ebenfalls durch das Material einer Decklage 6 verkleidet, die um die Seitenkanten der Versteifungseinlage herumgeführt und durch Steppnähte fixiert ist.

Die in Fig. 4 dargestellte Ausführungsform des Stirnbandes umfasst ebenfalls eine Versteifungseinlage 4 und eine auf der nach außen weisenden Seite des Stirnbandes vorgesehene Vliesmateriallage 8, die hier um den Seitenkantenbereich der Versteifungseinlage 4 herumgeführt ist, und auf Seiten der Polsterung 5 gemeinsam mit einer Decklage 6 fixiert ist. Die Fixierung erfolgt vorzugsweise über stark vergrößert angedeutete Steppnähte 9.

In Fig. 5 ist in teilweise aufgebrochener Darstellung ein Abschnitt eines erfindungsgemäßen Stirnbandes gezeigt. Das hier dargestellte Stirnband umfaßt wiederum die flexible Versteifungseinlage 4, die das Stirnband gegen eine Biegung um eine Achse senkrecht zur Stirnbandauflagefläche aussteift. Die flexible Lage 4 ist zwischen die rückseitige Decklage, die hier beispielsweise durch das Vliesmaterial 8 gebildet ist, und die innenseitige Decklage 6 unter Zwischenlage des Polsterkörpers 5 eingebettet. Vorzugsweise durch einen Thermoschweißvorgang können auf der Innenseite des Stirnbandes Steppstellen 10 ausgebildet werden, durch welche der Polsterung eine gewisse Vorspannung verliehen werden kann. Die Steppstellen können beispielsweise im Rahmen eines Ultraschallschweißvorganges oder durch ein entsprechend beheiztes Stempetwerkzeug ausgebildet werden. Eine unter fertigungstechnischen Gesichtspunkten besonders preiswerte Ausführungsform des Stirnbandes ist dadurch gegeben, dass die beiden Lagen 6, 8, ggf. auch die Versteifungseinlage 4, ebenfalls durch einen Thermoschweißvorgang oder durch einen Klebevorgang miteinender verbunden sind.

Eine bevorzugte Ausführungsform der Steppabschnitte ist in Fig. 6 dargestellt. Wie erkennbar, ist hier die Decklage 6 durch das heißgeschmolzene Material der Polsterung 5 auf die Versteifungslage 4 im Bereich der Schweißstellen 10 aufgeschweißt.

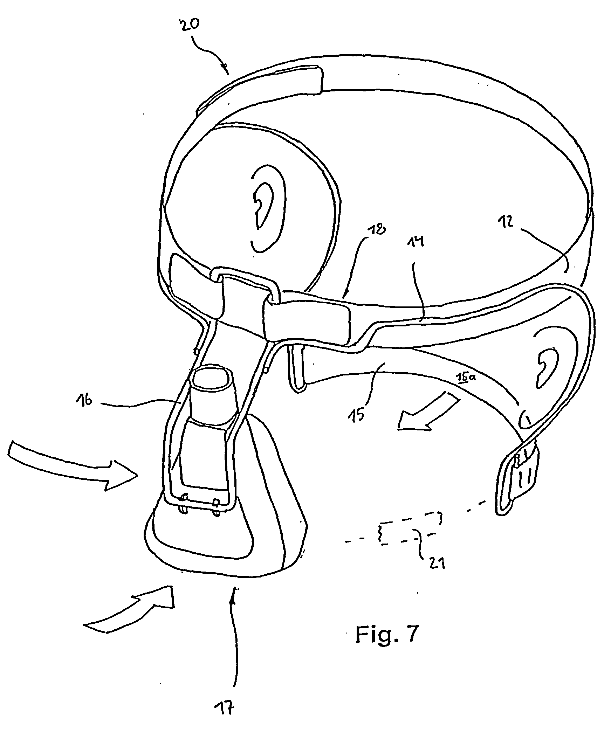

In Fig. 7 ist eine weitere bevorzugte Ausführungsform einer Halterung für eine Atemmaske dargestellt, die hier ein Stirnband 12 aufweist, das vorzugsweise in gleicher Weise wie das vorangehend beschriebene Stirnband 1 mit einer integralen Versteifungslage versehen ist. Das Stirnband 12 ist hier mit einem biegesteifen Element 14 versehen, das sich in Applikationsposition der Halterung bügelarfig abfallend hinter den Ohrmuscheln des Patienten zum Nackenbereich hin erstreckt. Das biegesteife Element 14 kann über eine Abstützeinrichtung 15 im Hinterkopf oder Nackenbereich des Patienten abgestützt werden. Bei der hier dargestellten Ausführungsform der Erfindung ist die Abstützeinrichtung durch ein längenverstellbares Bandelement 15 gebildet, das unterhalb des Ohrbereichs des Patienten mit dem biegesteifen Element 14 gekoppelt ist. An dem biegesteifen Element 14 ist eine Maskenfixiereinrichtung 16 vorgesehen, die sich vom Stirnbereich ausgehend zur Nasenspitze des Patienten hin erstreckt. Die Maskenfixiereinrichtung 16 ist mit einer Atemmaske 17 in einem Abschnitt gekoppelt, der im wesentlichen im Bereich des Kräfteschwerpunkts, der durch das Dichtkissen der Atemmaske definierten Gesichtsauflagezone verläuft. Bei der hier dargestellten Ausführungsform ist die Koppelung der Atemmaske 17 mit der Maskenfixiereinrichtung 16 derart vorgenommen, dass die Atemmaske in einem gewissen Bereich gekippt werden kann. Vorzugsweise ist die Anordnung derart abgestimmt, dass die Wirkungslinie der auf die Atemmaske 17 wirkenden Anpresskraft im wesentlichen mit der Wirkungslinie der durch die Abstützeinrichtung 15a und das Stirnband 12 selbst hervorgerufenen resultierenden Kraft entspricht.

Das biegesteife Element 14 ist vorzugsweise über eine Klettverschlußeinrichtung 18 an dem Stirnband 12 lösbar fixiert.

Die Maskenfixiereinrichtung 16 sowie das biegesteife Element 14 sind bei der hier dargestellten Ausführungsform aus einem Federstahldraht gefertigt.

Das Stirnband 12 weist weiterhin eine Schnellverschlußeinrichtung 20 auf, über welche das Stirnband 12 definiert geweitet und verkürzt werden kann. Bei der hier dargestellten Ausführungsform ist es möglich, an dem biegesteifen Element 14 ein weiteres Bandelement (hier nur andeutungsweise dargestellt) anzubringen, welches ggf. auch in der Art einer unteren Gurtbandanordnung zusätzliche Haltekräfte auf die Atemmaske 17 aufgebracht werden können.

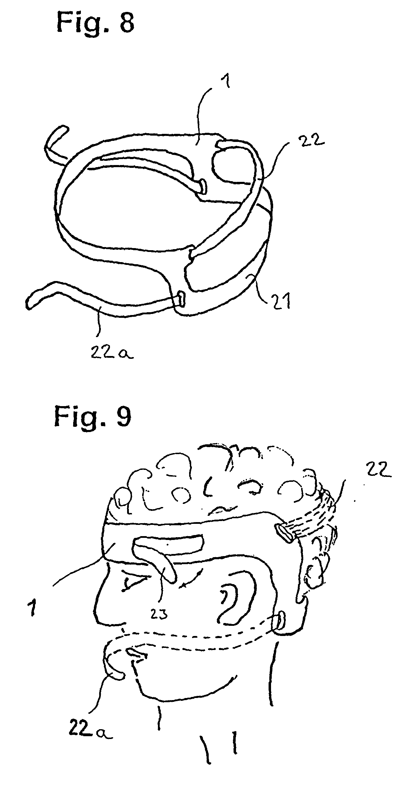

In Fig. 8 ist eine weitere Ausführungsform einer Halterung für eine Atemmaske dargestellt, die hier wiederum ein Stirnband 1 aufweist, das mit einem in der Applikationsfläche weitgehend seitlich, biegesteifen, jedoch in Umfangsrichtung abwickelbaren Versteifungseinlage 4 versehen ist. Das Stirnband 1 weist hier einen in Applikationsposition hinter dem Ohrbereich des Patienten abfallenden und sich anschließend um den Nackenbereich herum erstreckenden Abschnitt 21 auf. Zur weiteren Fixierung des Stirnbandes 1 ist ein elastisches Band 22 vorgesehen, das sich um den Hinterkopfbereich des Patienten erstreckt. An dem in Applikationsposition auf der Stirn des Patienten aufliegenden Abschnitt des Stirnbandes 1 kann über Klettverschlussverbindungseinrichtungen ein Stirnauflageelement einer Atemmaskenanordnung fixiert werden. Über weitere untere Gurtbänder 22 können weitere Kräfte auf die Atemmaske aufgebracht werden.

In Fig. 9 ist beispielhaft eine Halterung für eine Atemmaske in Applikationsposition dargestellt, die in ihrem Aufbau im wesentlichen der in Fig. 8 dargestellten Halterung entspricht. die Halterung 1 erstreckt sich vom Stirnbereich des Patienten hin zu dessen oberen Ohrbereich und fällt unmittelbar hinter den Ohren des Patienten zum Nacken des Patienten nach unten hin ab. über die untere Gurtbandanordnung kann eine nicht näher dargestellte Atemmaske zusätzlich fixiert werden. Durch die Fixierung der Atemmaske über das Stirnauflageelement und die untere Gurtanordnung 22 wird eine unter ergonomischen Gesichtspunkten vorteilhafte Applikation der Atemmaske erreicht. Das Stirnauflageelement der Atemmaske (nicht dargestellt) kann durch einen hier vereinfacht dargestellten Klettverschluß 23 erfolgen.

Anstelle des bei der Ausführungsform gem. Fig. 8 vorgesehenen elastischen Zuges 22 ist es auch möglich, die Halterung 1 derart auszubilden, dass diese im Hinterkopfbereich des Patienten eine größere Öffnung definiert und sich hierbei über wenigstens zwei Hinterkopfabschnitte 23, 24 im Hinterkopfbereich des Patienten abstützt. Die Hinterkopfabschnitte 23, 24 sind vorzugsweise wie hier dargestellt, längenverstellbar ausgebildet.

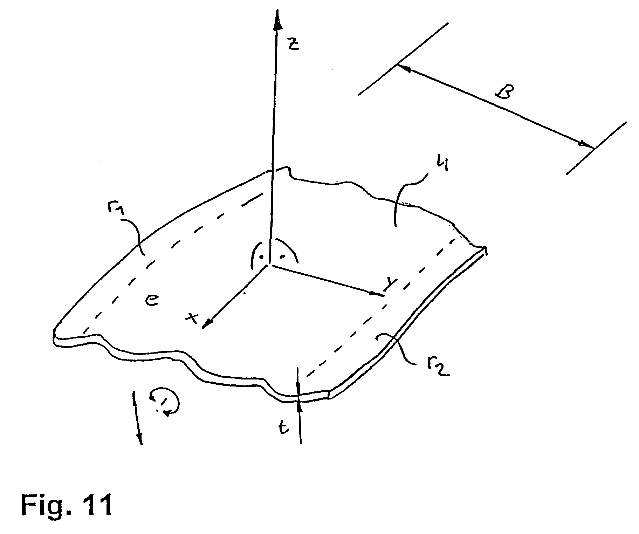

In Fig. 11 ist eine Prinzipskizze zur Erläuterung der mechanischen Eigenschaften der flexiblen Versteifungseinlage 4 dargestellt. Die flexible Versteifungseinlage erlaubt eine Biegeverformung, um die in der Hauptebene e der Versteifungseinlage 4 verlaufenden Hauptachsen x und y. Verformungen der Versteifungseinlage 4 um die senkrecht zur Ebene e verlaufende Hauptachse z werden aufgrund der großen Breite der Versteifungseinlage weitgehend vermieden. Die Breite B der Versteifungseinlage 4 sowie die Dicke t der Versteifungseinlage 4 und der E-Modul E der Versteifungseinlage 4 sind derart abgestimmt, dass die bei der Applikation einer Atemmaske auftretenden Biegemomente um die Hauptachse Z keine unzulässig hohen Verformungen verursachen.

Da die Biegesteifigkeit der Versteifungslage 4 um die Hauptachse Z im wesentlichen durch den Randzonenbereich r1, r2 der Versteifungseinlage 4 bestimmt ist, ist es möglich, im Zwischenbereich Öffnungen vorzusehen, so dass die Versteifungseinlage 4 eine im wesentlichen fachwerkartige Struktur aufweist.

Eine fachwerkartige Struktur der Versteifungseinlage 4 kann insbesondere bei der Herstellung der Versteifungseinlage 4 durch ein Kunststoffspritzwerkzeug realisiert werden.

In Zonen besonders hoher Biegebelastung ist es auch möglich, mehrere Versteifungseinlagen in dem Stirnband vorzusehen, oder die Versteifungseinlagen entsprechend lokal verdickt auszubilden.

## Patentansprüche

1. Halterung für eine Atemmaske mit einem Stirnbandabschnitt, der in Applikationsposition sich vom Stirnbereich des Patienten bis in den hinter den Ohren liegenden Hinterkopfbereich des Patienten erstreckt, **dadurch gekennzeichet, dass** der Stirnbandabschnitt wenigstens eine flexible und seitlich weitgehend biegesteife Versteifungseinlage (4) aufweist, die eine Biegeverformung um die in einer Hauptfläche (e) der Versteifungseinlage (4) verlaufenden Hauptachsen (X, Y) erlaubt und Verformungen um eine senkrecht zur Hauptfläche (e) verlaufende Hauptachse (Z) weitgehend vermeidet, zum Aussteifen des Stirnbandabschnittes in Seitenrichtung.

2. Halterung nach Anspruch 1, **dadurch gekennzeichnet, dass** die flexible Versteifungseinlage (4) aus einem Kunststoffflachmaterial gebildet ist.

3. Halterung nach Anspruch 1 **dadurch gekennzeichnet, dass** die flexible Versteifungseinlage (4) aus einem Blechstreifen gebildet ist.

4. Halterung nach Anspruch 1 oder 2 **dadurch gekennzeichnet, dass** die flexible Versteifungseinlage (4) aus einem thermoanformbaren Material gebildet ist.

5. Halterung nach wenigstens einem der Ansprüche 1-4, **dadurch gekennzeichnet, dass** der Stirnbandabschnitt mit einer Polstereinrichtung (5,6,8) versehen ist.

6. Halterung nach wenigstens einem der Ansprüche 1-5, **dadurch gekennzeichnet, dass** der Stirnbandabschnitt mit einem Klettverschluss-Vliesmaterial versehen ist.

7. Halterung nach wenigstens einem der Ansprüche 1-6, **dadurch gekennzeichnet, dass** das Stirnband mehrlagig kaschiert ausgebildet ist.

8. Halterung nach wenigstens einem der Ansprüche 1-7, **dadurch gekennzeichnet, dass** die flexible Versteifungseinlage (4) mit Durchbrüchen versehen ist.

9. Halterung nach wenigstens einem der Ansprüche 1-8, **dadurch gekennzeichnet, dass** der Stirnbandabschnitt in Applikationsposition einen vom Stirnbereich des Patienten zum oberen Ohrbereich gerichteten Verlauf und im Ohrbereich einen lokal hochgezogenen und im Hinterkopfbereich wieder abfallenden Verlauf aufweist.

10. Halterung nach wenigstens einem der Ansprüche 1-9, **dadurch gekennzeichnet, dass** die Polsterung des Stirnbandes durch eine Schaumstoffeinlage gebildet ist, die sich in Applikationsposition des Stirnbandes zwischen der flexiblen Versteifungseinlage und dem Patienten befindet.

11. Halterung für eine Atemmaske nach Anspruch 10, **dadurch gekennzeichnet, dass** die Polsterung abgesteppte Abschnitte aufweist.

12. Halterung für eine Atemmaske nach Anspruch 10 oder 11, **dadurch gekennzeichnet, dass** die abgesteppten Abschnitte durch einen Thermoschweißvorgang ausgebildet sind.

## Claims

1. A holding arrangement for a respiration mask comprising a headband portion which in an application position extends from the forehead region of the patient into the region of the back of the head of the patient which is behind the ears, **characterised in that** the headband portion has at least one flexible and laterally substantially flexurally stiff reinforcing insert (4) which allows bending deformation about the main axes (X, Y) extending in a main surface (e) of the reinforcing insert (4) and substantially prevents deformations about a main axis (Z) extending perpendicularly to the main surface (e), for stiffening the headband portion in the lateral direction.

2. A holding arrangement according to claim 1 **characterised in that** the flexible reinforcing insert (4) is formed from a flat plastic material.

3. A holding arrangement according to claim 1 **characterised in that** the flexible reinforcing insert (4) is formed from a sheet metal strip.

4. A holding arrangement according to claim 1 or claim 2 **characterised in that** the flexible reinforcing insert (4) is formed from a thermo-formable material.

5. A holding arrangement according to at least one of claims 1 to 4 **characterised in that** the headband portion is provided with a padding means (5, 6, 8).

6. A holding arrangement according to at least one of claims 1 to 5 **characterised in that** the headband portion is provided with a touch-and-close fastener non-woven material.

7. A holding arrangement according to at least one of claims 1 to 6 **characterised in that** the headband is of a multi-layer lined configuration.

8. A holding arrangement according to at least one of claims 1 to 7 **characterised in that** the flexible reinforcing insert (4) is provided with openings therethrough.

9. A holding arrangement according to at least one of claims 1 to 8 **characterised in that** the headband portion in the application position is of a configuration directed from the forehead region of the patient to the upper ear region and in the ear region it is of a configuration which is locally raised and falls again in the region of the back of the head.

10. A holding arrangement according to at least one of claims 1 to 9 **characterised in that** the padding of the headband is formed by a foam insert which in the application position of the headband is between the flexible reinforcing insert and the patient.

11. A holding arrangement for a respiration mask according to claim 10 **characterised in that** the padding has stitched portions.

12. A holding arrangement for a respiration mask according to claim 10 or claim 11 **characterised in that** the stitched portions are formed by a thermal welding operation.

## Revendications

1. Système de retenue pour masque respiratoire comportant une section de serre-tête qui dans la position d'application s'étend à partir de la zone du front du patient jusqu'à la zone de l'arrière de la tête se trouvant derrières les oreilles du patient, **caractérisé en ce que** la section de serre-tête présente au moins un insert de rigidification flexible (4) et latéralement largement résistant à la flexion, qui permet une déformation par flexion autour des axes principaux (X, Y) s'étendant dans une surface principale (e) de l'insert de rigidification (4) et qui évite largement des déformations autour d'un axe principal (Z) s'étendant verticalement par rapport à la surface principale (e) pour rigidifier la section de serre-tête en direction latérale.

2. Système de retenue selon la revendication 1, **caractérisé en ce que** l'insert de rigidification flexible (4) est formé en un matériau plat en plastique.

3. Système de retenue selon la revendication 1, **caractérisé en ce que** l'insert de rigidification flexible (4) est formé dans une bande de tôle.

4. Système de retenue selon les revendications 1 ou 2, **caractérisé en ce que** l'insert de rigidification flexible (4) est formé dans un matériau thermomoulable.

5. Système de retenue selon au moins une des revendications 1 à 4, **caractérisé en ce que** la section de serre-tête est pourvue d'un dispositif de rembourrage (5, 6, 8).

6. Système de retenue selon au moins une des revendications 1 à 5, **caractérisé en ce que** la section de serre-tête est formée en matière non tissée à fermeture Velcro.

7. Système de retenue selon au moins une des revendications 1 à 6, **caractérisé en ce que** le serre-tête est formé en plusieurs couches contrecollées.

8. Système de retenue selon au moins une des revendications 1 à 7, **caractérisé en ce que** l'insert de rigidification flexible (4) est pourvu de perforations.

9. Système de retenue selon au moins une des revendications 1 à 8, **caractérisé en ce que** la section de serre-tête présente dans la position d'application une évolution s'éloignant de la zone du front du patient vers la partie supérieure de l'oreille, dans la zone de l'oreille une évolution localement relevée et dans la zone de l'arrière de la tête de nouveau une évolution vers le bas.

10. Système de retenue selon au moins une des revendications 1 à 9, **caractérisé en ce que** le rembourrage du serre-tête est formé par un insert mousse qui dans la position d'application du serre-tête se trouve entre l'insert de rigidification flexible et le patient.

11. Système de retenue pour masque respiratoire selon la revendication 10, **caractérisé en ce que** le rembourrage présente des sections surpiquées.

12. Système de retenue pour masque respiratoire selon les revendications 10 ou 11, **caractérisé en ce que** les sections surpiquées sont formées par un procédé de soudure à chaud.
